# FASCICULE DE BREVET EUROPEEN

(11) **EP 0 835 300 B1**
(45) Date de publication et mention de la délivrance du brevet: **24.07.2002**
(21) Numéro de dépôt: 96911026.1
(22) Date de dépôt: 03.04.1996
(51) Int. Cl.: C12M 1/26

(54) **DISPOSITIF DE CONTROLE MICROBIOLOGIQUE D'UN GAZ SOUS PRESSION**
ANLAGE FÜR DIE MIKROBIOLOGISCHE UNTERSUCHUNG VON DRUCKGAS
MICROBIOLOGICAL PRESSURISED GAS CONTROL DEVICE

(30) Priorité: 06.04.1995 FR 9504107
(43) Date de publication de la demande: 15.04.1998
(73) Titulaire: Ultra Propre Nutrition Industrie Recherche (U.N.I.R.), 75008 Paris (FR)
(72) Inventeur: CHEVALIER, Philippe, décédé (FR)
(74) Mandataire: Warcoin, Jacques
(86) Numéro de dépôt international: FR9600506
(87) Numéro de publication internationale: WO9631594

(56) Documents cités:
- EP-A- 0 450 850
- GB-A- 2 224 118
- US-A- 2 894 877
- US-A- 4 255 172
- US-A- 4 274 846
- FOOD TECHNOLOGY, vol. 35, no. 2, CHICAGO US, page 103 XP002008157 SPIRAL SYSTEM INSTRUMENTS, INC.: "Air Sampling Instrument."

## Description

La présente invention concerne un dispositif de contrôle microbiologique d'un gaz sous pression.

L'invention trouve une application particulièrement avantageuse pour contrôler par exemple de l'air ou de l'oxygène comprimé qui circule dans un réseau, tel qu'un réseau désservant des blocs opératoires ou salles de soins d'hôpitaux, ou bien des salles propres ou blanches utilisées dans l'industrie pharmaceutique et cosmétique et dans l'industrie agro-alimentaire.

On connait déjà des dispositifs de contrôle microbiologique de l'air ambiant, utilisant le principe de l'impaction.

Dans ces dispositifs connus, une boite de Pétri disposée en regard d'une plaque d'impaction percée de nombreux petits orifices, dénommée par la suite "tamis", permet de recueillir les poussières contenues dans l'air ambiant aspiré par une pompe à travers les perforations du tamis, les jets traversant les perforations du tamis étant projetés sur le milieu nutritif de la boite de Pétri.

Les micro-organismes contenus dans les poussières impactées par les jets de gaz sur le milieu nutritif, se multiplient et donnent naissance à des colonies si le milieu nutritif leur convient. Au bout de quelques jours d'incubation, ces germes sont donc remplacés par des colonies visibles dont certaines sont identifiables à l'oeil nu.

Le contrôle de gaz sous pression à l'aide des dispositifs connus impose une détente préalable du gaz initialement comprimé à plusieurs bars ce qui conduit classiquement à un jet sonique suivi d'une onde de choc. Ces variations brutales de pression du gaz provoque la destruction des micro-organismes contenus dans les poussières portées par le gaz. Les mesures sur le gaz détendu ne sont donc pas des mesures représentatives de la biocontamination dudit gaz.

La présente invention propose un nouveau dispositif de contrôle microbiologique d'un gaz sous pression chargé en poussières sans provoquer la destruction totale ou partielle des micro-organismes contenus dans lesdites poussières.

Plus particulièrement, le dispositif conforme à l'invention, comporte les caractéristiques de la revendication 1.

Ainsi, un tel dispositif peut de façon avantageuse être raccordé directement sur le réseau de distribution du gaz comprimé, ou bien sur la sortie d'un récipient contenant un tel gaz.

Selon une caractéristique avantageuse du dispositif conforme à l'invention, il comporte en aval des moyens de collecte, une tuyère sonique positionnée à la sortie de ladite cavité.

Une telle tuyère sonique permet de détendre le gaz, sous pression dans ledit dispositif, pour l'amener à la pression atmosphérique, tout en imposant un débit volumique constant. Ceci est avantageux, car quelque soit la pression, le dispositif selon l'invention permet d'obtenir un débit volumique identique au niveau de l'impaction.

Bien entendu, en fonction du débit volumique désiré on peut adapter le diamètre de la tuyère sonique.

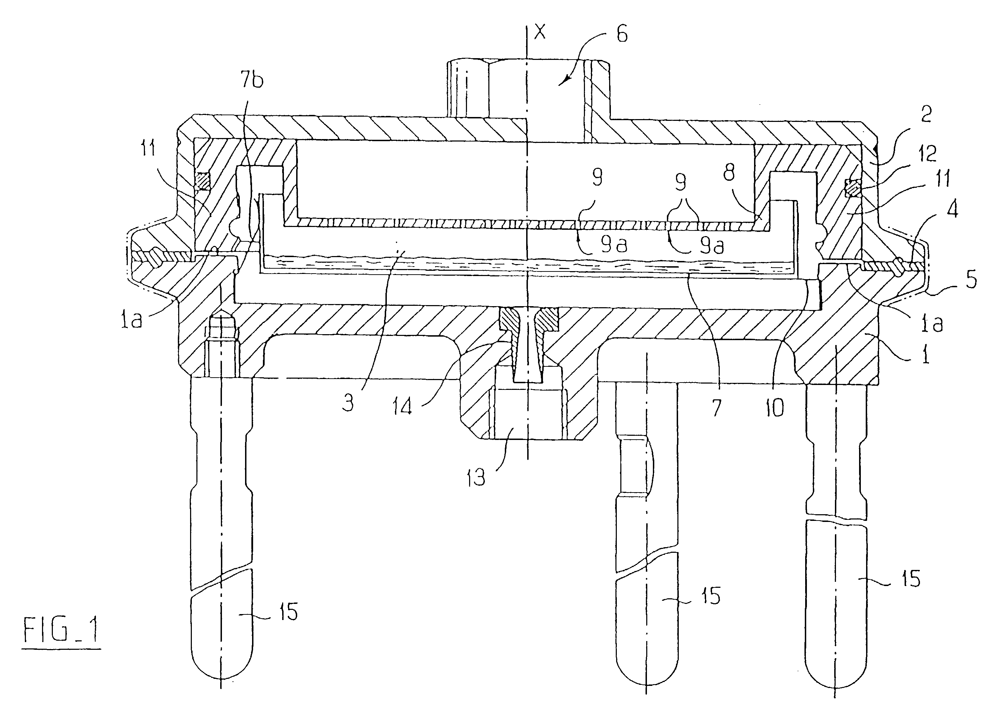

La description qui va suivre en regard du dessin annexé donné à titre d'exemple non limitatif, fera bien comprendre en quoi consiste l'invention et comment elle peut être réalisée.
La figure unique est une vue en coupe axiale, d'un dispositif de contrôle microbiologique de gaz sous pression, conforme à l'invention.

Sur la figure, on a représenté un dispositif de contrôle microbiologique d'un gaz sous pression, tel que par exemple, de l'air comprimé ou bien de l'oxygène comprimé.

Ce dispositif comporte un boîtier 1 fermé par un couvercle 2, de façon à définir une cavité intérieure 3 essentiellement de révolution autour d'un axe central X. Le couvercle 2 est fermé de manière étanche sur le boîtier 1 par l'intermédiaire d'un joint annulaire 4 et d'un collier de serrage classique 5. Le couvercle 2 comporte une ouverture 6 centrée sur l'axe X de la cavité. Dans cette ouverture 6 est positionnée une vanne (ici non représentée) apte à être raccordée par exemple à un réseau de distribution d'un gaz sous pression. L'ouverture de cette vanne permet l'entrée de gaz dans ladite cavité 3.

En outre, le boîtier 1 comporte des aménagements intérieurs permettant de positionner perpendiculairement à l'axe X, en regard de l'ouverture 6, une boite de Pétri 7 de type standard de diamètre de 90 mm par exemple et au-dessus de la boite de Pétri 7 une plaque d'impaction perforée 8 appelée par la suite "tamis". La boite de Pétri 7 est remplie d'environ 16 ml de milieu nutritif , appelée gélose. La distance entre les sorties 9a des perforations 9 du tamis 8 et la surface de la gélose est déterminée en fonction de la dimension d des perforations 9a.

Les perforations 9 sont réalisées dans le tamis 8 de telle sorte que l'axe des perforations 9 est dirigé essentiellement de manière perpendiculaire au fond de la boîte de Pétri 7. Les perforations 9 débouchent en direction du fond de la boîte de Pétri 7. Les dimensions desdites perforations 9 et le nombre de perforations 9 du tamis 8 sont déterminées en fonction de la vitesse désirée du jet de gaz traversant chaque perforation. Cette vitesse dépend du diamètre des poussières que l'on désire collecter dans la boîte de Pétri.

Pour collecter des poussières aussi fines que possible, il est souhaitable que les jets de gaz entraînant lesdites poussières, prennent à la sortie des perforations 9 du tamis 8 un virage très serré, ce qui impose des trous de petits diamètres. Ainsi, les jets de gaz comprimé sortant desdites perforations 9 s'épanouissent sur le milieu nutritif de la boîte de Pétri 7, selon un trajet présentant un très faible rayon de courbure, et à grande vitesse de sorte que ces poussières fines soient projetées directement sur le milieu nutritif de la boîte de Pétri 7. Toutefois, il ne faut pas que la vitesse de projection des poussières sur le milieu nutritif de la boite de Pétri 7 soit trop grande car les micro-organismes contenus dans les poussières risquent d'être stressés par l'impact violent sur le milieu nutritif et de ce fait, présenter des difficultés à se reproduire et à former une colonie dans le milieu nutritif.

Pour ces raisons précitées, la vitesse préconisée des jets de gaz comprimé sortant des perforations du tamis est de l'ordre de 20 mètres par seconde. Ce qui impose pour le tamis 8, une perforation d'environ 600 trous de 0,4 mm de diamètre pour obtenir un débit d'environ 100 litres par minute. Le diamètre du tamis 8 est sensiblement égal à celui de la boîte de Pétri ici 90 mm. La distance h entre la surface de la gélose et le tamis 8 est compris environ entre 2d et 6d.

Selon le mode de réalisation représenté, la boîte de Pétri 7 est positionnée sur des pattes de support d'un ressort à lame 10 (ici trois pattes réparties sur la circonférence de la boite de Pétri 7 dont une seule est représentée) placé sur le fond de la cavité 3 formé par le boîtier 1. Le tamis 8 est suspendu à un support 11 (formant ici une seule pièce avec ledit tamis) positionné sur une partie en décrochement la du boîtier 1. Le ressort à lame 10 comporte des pattes de centrage 7b (dont une seule est représentée) qui s'étendent transversalement à l'axe X en direction du support 11 pour venir en appui contre ce dernier. L'étanchéité entre ce support 11 et le couvercle 2 est réalisée par un joint torique 12.

Il est intéressant de préciser que le volume de la cavité intérieure 3 se situant en-dessous des moyens de collecte formés par le tamis 8 et la boîte de Pétri 7 soit minimum. Dans l'exemple représenté, ce volume est égal à environ 30 cm³. Par contre, le volume de la cavité compris entre le tamis 8 et l'entrée 6 de la cavité 3 n'est pas déterminant.

La cavité 3 comprend une sortie de gaz 13 vers l'extérieur prévue dans le boîtier 1. Cette sortie 13 est centrée sur l'axe X. Une tuyère sonique 14 est positionnée en aval de la boîte de Pétri 7 et en amont de la sortie de gaz 13. Plus particulièrement, la tuyère sonique 14 est positionnée selon l'axe X dans un aménagement intérieur du boîtier 1 et débouche directement dans la sortie de gaz 13.

Selon le mode de réalisation représenté, le boîtier 1 est positionné sur des pieds 15.

Un tel dispositif fonctionne de la manière suivante.

L'ouverture 6 du couvercle 2 est raccordée à un distributeur de gaz sous pression de telle sorte que l'ouverture de la vanne provoque l'entrée du gaz sous pression dans la cavité 3. Ce gaz sous pression traverse alors le tamis 8 par les perforations 9 et projette à la sortie du tamis 8 les poussières qu'il contient sur le milieu nutritif de la boîte de Pétri 7, puis s'échappe sur les côtés de la boîte de Pétri 7. Le gaz sous pression s'évacue alors vers l'extérieur via la tuyère sonique 14. Lorsque le dispositif fonctionne, la pression régnant dans la cavité intérieure 3 est alors égale à la pression du gaz sous pression la traversant. La tuyère sonique 14 permet de détendre le gaz arrivant sous pression, jusqu'à la pression atmosphérique, tout en imposant un débit volumique constant après son amorçage et notamment un débit volumique constant au niveau de l'impaction des poussières sur le milieu nutritif. Ceci est avantageux car c'est ce débit volumique qui détermine les propriétés de l'impaction.

Les poussières récoltées à l'aide de ce dispositif ont un diamètre supérieur à 0,5 µm.

Le débit de gaz d'un tel dispositif peut varier entre environ 10 et 1000 litres par minute, en fonction du nombre et du diamètre des trous du tamis.

On pourra envisager d'autres moyens de fixation de la boîte de Pétri que le ressort à lame et les pattes d'accrochage, tel que par exemple des crochets de suspension montés dans la paroi interne du boîtier. En outre, on peut envisager de réaliser certaines pièces assemblées du dispositif de façon monobloc.

## Revendications

1. Dispositif de contrôle micro biologique d'un gaz sous pression chargé en poussières, comprenant :
- une cavité intérieure (3) munie d'une entrée (6) et d'une sortie de gaz (13), et
- des moyens de collecte (7,8) placés dans la cavité pour accueillir les poussières,
**caractérisé en ce que** le dispositif est agencé de sorte que, lorsque l'entrée reçoit un gaz sous pression à une pression supérieure à la pression atmosphérique et que la sortie (13) est à la pression atmosphérique, il règne dans la cavité au niveau des moyens de collecte (7,8) une pression égale à la pression à l'entrée (6) de la cavité.

2. Dispositif selon la revendication 1, **caractérisé en ce qu'**il comporte en aval des moyens de collecte (7, 8), une tuyère sonique (14) positionnée à la sortie de gaz (13) de ladite cavité (3).

3. Dispositif selon l'une des revendications 1 ou 2, caractérisé en ce le volume de la cavité (3), compris entre le moyen de collecte (7) et la sortie de gaz (13) est minimum et de l'ordre de 30 cm³.

4. Dispositif selon l'une quelconque des revendications 1 à 3, **caractérisé en ce qu'**il comprend un boîtier (1) fermé de manière étanche par un couvercle (2) de façon à définir ladite cavité intérieure (3), ledit boîtier (1) étant pourvu d'aménagements intérieurs (10, 11) pour porter lesdits movens de collecte (7, 8).

5. Dispositif selon la revendication 4, **caractérisé en ce que** lesdits moyens de collecte comprennent une boite de Pétri (7) positionnée dans ladite cavité fermée (3), contenant un milieu nutritif en regard de l'entrée (6) de ladite cavité (3), et une plaque d'impaction perforée (8) placée au-dessus de la boite de Pétri (7) parallèlement à cette dernière.

6. Dispositif selon la revendication 5, **caractérisé en ce que** la plaque d'impaction (8) comporte environ 600 perforations (9) débouchant en direction du fond de la boîte de Pétri (7), lesdites perforations (9) présentant un diamètre (d) d'environ 0,4 mm.

7. Dispositif selon l'une des revendications 5 ou 6, **caractérisé en ce que** la surface du milieu nutritif placé dans la boite de Pétri (7) et la plaque d'impaction (8) sont placées à une distance h l'une de l'autre, qui est fonction des dimensions des perforations (9) de la plaque d'impaction (8).

## Patentansprüche

1. Vorrichtung zur mikrobiologischen Kontrolle eines unter Druck stehenden, mit Stäuben beladenen Gases, umfassend:
- einen inneren Hohlraum (3), der mit einem Einlass (6) und einem Gasauslass (13) ausgestattet ist, und
- Sammelmittel (7,8), die in dem Hohlraum angeordnet sind, um die Stäube aufzunehmen,
**dadurch gekennzeichnet, dass** die Vorrichtung dergestalt eingerichtet ist, dass, wenn der Einlass ein unter Druck stehendes Gas bei einem Druck über Atmosphärendruck aufnimmt und der Auslass (13) sich bei Atmosphärendruck befindet, in dem Hohlraum auf dem Niveau der Sammelmittel (7,8) ein Druck herrscht, der gleich dem Druck am Einlass (6) des Hohlraums ist.

2. Vorrichtung nach Anspruch 1, **dadurch gekennzeichnet, dass** sie stromabwärts von den Sammelmitteln (7,8) eine Schalldüse (14), die sich am Gasauslass (13) des Hohlraums (3) befindet, umfasst.

3. Vorrichtung nach einem der Ansprüche 1 oder 2, **dadurch gekennzeichnet, dass** das Volumen des Hohlraums (3) zwischen dem Sammelmittel (7) und dem Gasauslass (13) minimal und von der Größenordnung von 30 cm³ ist.

4. Vorrichtung nach einem der Ansprüche 1 bis 3, **dadurch gekennzeichnet, dass** sie ein Gehäuse (1) umfasst, das durch eine Verschlusskappe (3) dicht verschlossen ist, wodurch der innere Hohlraum (3) definiert wird, wobei das Gehäuse (1) mit inneren baulichen Einrichtungen (10,11) versehen ist, um die Sammelmittel (7,8) zu tragen.

5. Vorrichtung nach Anspruch 4, **dadurch gekennzeichnet, dass** die Sammelmittel eine in dem geschlossenen Hohlraum (3) angeordnete Petrischale (7), die ein Nährmedium gegenüber dem Einlaas (6) des Hohlraums (3) enthält, und eine perforierte Prallplatte (8), die oberhalb der Petrischale (7) parallel zu dieser letzteren angeordnet ist, umfassen.

6. Vorrichtung nach Anspruch 5, **dadurch gekennzeichnet, dass** die Prallplatte (8) ungefähr 600 Perforationen (9), die in Richtung des Bodens der Petrischale (7) münden, umfasst, wobei die Perforationen (9) einen Durchmesser (d) von ungefähr 0,4 mm aufweisen.

7. Vorrichtung nach einem der Ansprüche 5 oder 6, **dadurch gekennzeichnet, dass** die Oberfläche des in die Petrischale (7) eingefüllten Nährmediums und die Prallplatte (8) zueinander in einem Abstand h angeordnet sind, der von den Abmessungen der Perforationen (9) der Prallplatte (8) abhängig ist.

## Claims

1. Microbiological control device for a pressurised gas carrying dust particles comprising:
- an internal cavity (3) fitted with a gas inlet (6) and a gas outlet (13), and
- collections means (7, 8) set in the cavity to receive said dust,
**characterised in that** the device is adjusted such that, when the inlet receives a pressurised gas at a pressure higher than atmospheric pressure and when the outlet (13) is at atmospheric pressure, the prevailing pressure in the cavity at the collection means (7, 8) is equal to the pressure at the inlet (6) of the cavity.

2. Device according to claim 1, **characterised in that** it comprises downstream from the collection means (7, 8), a sonic nozzle (14) placed at the gas outlet (13) of said cavity (3).

3. Device according to one or the other of claims 1 or 2, **characterised in that** the volume of the cavity (3), set between the collection means (7) and the gas outlet (13) is of minimum size and of the order of 30 cm³.

4. Device according to any one of claims 1 to 3, **characterised in that** it comprises a casing (1) closed in a sealed manner by a lid (2) so as to define said internal cavity (3), said casing (1) being provided with inner fittings (10, 11) to carry said collection means (7, 8).

5. Device according to claim 4, **characterised in that** said collection means comprise a Petri dish (7) placed in said closed cavity (3) containing a nutrient medium facing the inlet (6) of said cavity (3), and a perforated impact plate (8) set above the Petri dish (7) parallel to the latter.

6. Device according to claim 5, **characterised in that** the impact plate (8) comprises about 600 perforations (9) opening in the direction of the base of the Petri dish (7), said perforations (9) having a diameter of (d) of about 0.4 mm.

7. Device according to one or the other of claims 5 or 6, **characterised in that** the surface of the nutrient medium placed in the Petri dish (7) and the impact plate (8) are placed at a distance h from each other, which is a function of the dimensions of the perforations (9) of the impact plate (8).
